# EUROPEAN PATENT APPLICATION

(11) **EP 4 501 332 A1**
(43) Date of publication of application: **05.02.2025**
(21) Application number: 23306319.7
(22) Date of filing: 01.08.2023
(51) Int. Cl.: A61K 31/7024, A61K 33/26, A61K 9/00, A61P 7/08

(54) **DIALYSATE COMPOSITION COMPRISING IRON OXIDE NANOPARTICLES COATED WITH POLYMERIC COMPOUNDS**

(71) Applicant: Centre National de la Recherche Scientifique, 75016 Paris (FR); Université de Strasbourg, 67000 Strasbourg (FR); Hôpitaux Universitaires de Strasbourg (HUS), 67000 Strasbourg (FR); Institut National de la Santé et de la Recherche Médicale, 75013 Paris (FR)
(72) Inventor: BEGIN-COLIN, Sylvie, 67204 ACHENHEIM (FR); CHOQUET, Philippe, 68590 RORSCHWIHR (FR); ZALOSZYC, Ariane, 67000 STRASBOURG (FR); LUCANTE, Théo, 67000 STRASBOURG (FR); KRETZ, Manon, 67300 SCHILTIGHEIM (FR); DE LOS ANGELES RAMIREZ, Maria, 67000 STRASBOURG (FR); DUENAZ RAMIREZ, Paula, 78210 SAINT-CYR L'ECOLE (FR); FISCHBACH, Michel, 67150 GERTSHEIM (FR); CARTON, Anne, 67840 KILSTETT (FR)
(74) Representative: Novagraaf Technologies

(57) **Abstract**

The present invention relates to a dialysate composition having a composition having a composition allowing for a balance with blood comprising mineral salts and a pH buffer, and comprising at least one iron oxide nanoparticle (Fe²⁺ and/or Fe³⁺) coated with at least one polymeric compound.

## Description

The present invention concerns a dialysate composition having a composition allowing for a balance with blood, said composition comprising mineral salts, pH buffer and optionally at least one osmotic compound, and comprising at least one iron oxide nanoparticle (Fe²⁺/Fe³⁺) coated with at least one polymeric compound. Typically, the polymeric compound can act as a surfactant.

Chronic kidney disease (CKD), characterized by a progressive loss of kidney function over time, is currently a major health problem worldwide, with 75 million people suffering from CKD in Europe. This syndrome leads to the accumulation in the blood of uremic toxins and phosphate that are normally regularly eliminated by healthy kidneys, resulting in the development of toxic symptoms, persistent inflammation, oxidative stress and endothelial dysfunction, which eventually lead to major clinical complications, including cardiovascular disease and death. Patients suffering from end-stage renal failure therefore cannot survive without renal replacement therapy, i.e. transplantation or dialysis. Transplantation is the best option for patients, but the waiting list can be long and most patients require some form of dialysis while waiting for a transplant. Dialysis can be carried out in two ways: haemodialysis (HD) and peritoneal dialysis (PD).

Haemodialysis consists in purifying the blood via extracorporeal circulation, where the blood is filtered using a machine. In peritoneal dialysis, the process is different: the dialysate is introduced into the patient's peritoneal cavity and it is through the peritoneum that exchanges take place. By diffusion and osmosis mechanisms through the capillaries, toxins and excess water pass into the dialysate. The composition of the dialysate is balanced with that of the blood within a few hours.

Whilst dialysis remains an effective means of treating chronic kidney disease, current processes do not eliminate phosphates and toxins in sufficiently large quantities. This imbalance in phosphate in the blood leads to very high cardiovascular mortality and bone disorders in patients. In order to control the phosphataemia, patients with CKD must follow a restrictive diet, which can lead to malnutrition and greatly reduce their life quality or ingest phosphate binders, which are not always well tolerated or effective.

Thus, there is an urgent need to improve the treatment of chronic kidney disease, to control the accumulation of phosphate in blood, and to prevent hyperphosphatemia. Moreover, there is a need to find a better dialysate composition to capture phosphates from the blood more effectively and if possibly to improve also the removal of other toxins.

The present invention fulfills these needs.

Indeed, as shown in the examples, the inventors have discovered that formulating new dialysates by adding nanomaterials based on iron oxide nanoparticles improve the elimination of phosphate and potentially toxins from blood and biological fluids.

A first object of the present invention is to provide a dialysate allowing for a balance with blood, said composition comprising mineral salts and a pH buffer, and comprising at least one iron oxide nanoparticle (Fe²⁺ and/or Fe³⁺) coated with at least one polymeric compound. Preferably, the dialysate composition further comprises at least one osmotic compound.

Another object of the present invention is a composition for use as a medicament, more preferably for use as a dialysate for blood purification.

### Dialysate composition

A first object of the present invention is a dialysate composition allowing for a balance with blood, said composition comprising mineral salts, pH buffer and optionally at least one osmotic compound, and comprising at least one iron oxide nanoparticle (Fe²⁺ and/or Fe³⁺) coated with at least one polymeric compound.

By "dialysate composition", it is meant an aqueous composition comprising at least one mineral salt (also called "electrolyte"), a pH buffer and optionally at least one osmotic compound. The purpose of said dialysate composition is to pull the toxins and phosphates from the blood to the dialysate.

The dialysate composition of the invention is a composition that can be used in peritoneal dialysis and/or in hemodialysis.

Preferably, the dialysate composition according to the invention has a composition similar to that of blood.

By "composition similar to that of blood" it is meant that the composition comprises at least one mineral salt in a concentration similar to those of blood and a pH buffer. Optionally said composition further comprises at least one osmotic compound especially in a concentration that allows it to be in balance with the blood and allows exchanges by diffusion between the dialysate and the blood.

Specifically, the dialysate composition is prepared in such a way as to facilitate exchange by diffusion between the blood and the dialysate.

By "blood", it is meant liquids that are present in the human body. Preferably, the blood is human blood.

Preferably, the dialysate composition according to the invention comprises at least one mineral salt.

Preferably, the mineral salt is selected from the group consisting of calcium, phosphorus, potassium, sodium, chloride, magnesium, iron, zinc, iodine, chromium, copper, fluoride, molybdenum, manganese, sulphur, selenium and mixtures thereof.

Preferably, the mineral salt is selected from the group consisting of sodium, chloride, calcium, magnesium, potassium, sulphur and mixtures thereof. In a preferred embodiment, the mineral salt is selected among sodium, magnesium, potassium, calcium and mixtures thereof.

In a more preferred embodiment, the mineral salt is selected among sodium, potassium and mixtures thereof.

Typically, the amount of sodium in the dialysate composition is comprised between 120 and 150 mM. Typically, the amount of calcium in the dialysate composition is comprised between 1.2 and 1.9 mM. Typically, the amount of magnesium in the dialysate composition is comprised between 0.2 and 1.5 mM.

Typically, the amount of potassium in the dialysate composition is comprised between 0 and 5 mM, especially in the case of hemodialysis.

Typically, the amount of chloride in the dialysate composition is comprised between 90 and 110 mM, especially in the case of peritoneal dialysis.

Preferably, the dialysate composition according to the invention comprise at least one pH buffer. Preferably, the pH buffer is selected in the group consisting of acetate buffer, bicarbonate buffer, citrate buffer, lactate buffer and mixtures thereof.

More preferably, the pH buffer is selected in the group consisting of bicarbonate buffer, lactate buffer and mixtures thereof.

Typically, the amount of bicarbonate buffer in the dialysate composition is comprised between 0 and 45 mM.

Typically, the amount of lactate buffer in the dialysate composition is comprised between 0 and 40 mM, especially in the case of peritoneal dialysis.

Preferably, the pH of the dialysate composition is comprised between 4 and 8, preferably between 5 and 8 and more preferably between 5.5 and 7.5.

Preferably, the dialysate composition has an osmolarity or osmotic concentration comprised between 250 mOsm/L and 600 mOsm/L, more preferably between 250 mOsm/L and 350 mOsm/L.

By "osmolarity", it is meant a measure of the concentration of a solution, colloid or chemical compound, expressed as the number of "osmotically active" particles (osmoles) per litre. By "osmotically active" it is meant particles that are responsible for other properties of solutions, such as osmotic pressure, freezing point depression and water vapor pressure. These quantities are all linearly related to osmolality, making it possible to measure it.

The dialysate composition according to the invention may comprise at least one osmotic compound.

By "osmotic compound", it is meant an osmotically active ion or molecule in the peritoneal cavity and thereby forcing water retention intralumenally to maintain isotonicity with plasma. Preferably, the osmotic compound is selected from the group consisting of maltodextrin, sucrose, glucose, polyethylene glycol, mannitol, sorbitol, icodextrin, amino acids and mixtures thereof.

Preferably, the amino acid is selected in the group consisting of L-tyrosine, L-tryptophan, L-phenylalanine, L-threonine, L-serine, L-proline, glycine, L-alanine, L-valine, L-methionine, L-isoleucine, L-leucine, L-histidine, L-arginine, L-lysine (and its hydrochloride salt) and mixtures thereof.

Specifically, the dialysate composition that comprises at least one osmotic compound is mostly used for peritoneal dialysis.

### Iron oxide nanoparticles

According to the invention, the composition comprises at least one iron oxide nanoparticle (Fe²⁺/Fe³⁺) coated with at least one polymeric compound.

By 'iron oxide nanoparticle", it is meant a nanoparticle made up of an iron oxide derivative with one dimension being below 100 nm and having different shapes.

By "iron oxide derivative", it is meant a compound comprising at least iron and oxygen as constituting elements.

Iron oxides are a complex group of materials that have structure-dependent properties that are still not yet fully understood. Depending on how the nanoparticles are formed, their character may be one or more of the many different iron oxide species, including:
- Iron(II) oxide, wüstite (FeO),
- Iron(II,III) oxide, magnetite (Fe3 04),
- Iron(III) oxides (Fe2 O3) :
   ▪ alpha phase, hematite (α-Fe2 O3),
   ▪ beta phase, (β-Fe2 O3),
   ▪ gamma phase, maghemite (γ-Fe2 O3), or
   ▪ epsilon phase, (ε-Fe2 O3).
- Iron hydroxides
   ▪ Iron(III) oxide-hydroxide or ferric oxyhydroxide FeO(OH)
   ▪ Iron(II) hydroxide or ferrous hydroxide : Fe(OH)2
   ▪ Iron(III) hydroxide or ferric hydroxide : : Fe(OH)3
- Doped iron oxides : MxFe3-xO4 with M being a metallic element such as Al, Mn, Co, Zn, Y, In, Cr, or Nd.

Preferably, the iron oxide nanoparticles are made with magnetite or maghemite phase.

Typically, nanoscale magnetite is usually oxidized on its surface, so the composition of iron oxide nanoparticles usually consists of a magnetite core covered by an oxide layer mainly composed of maghemite phase. Such nanoparticles can also be oxidized after synthesis so that they consist only of the maghemite phase.

The iron oxide nanoparticles according to the invention are coated with at least one polymeric compound.

The polymeric compound should ensure the colloidal stability of nanoparticles in dialysates, an anchoring on the surface of nanoparticles weaker than that of phosphates, a biocompatibility and non-cytotoxicity (in the hypothesis that they can be desorbed during dialysis), a mean hydrodynamic size in the range 20-300 nm.

It should be added with an optimized amount to keep free adsorption sites. Their composition may favor the adsorption of toxins.

Specifically, the surface of the nanoparticles is functionalized by at least one polymeric compound.

Without being bound by any theory, the inventors have discovered that functionalizing these nanoparticles with a polymer or polymer mixture improves their stability in solution. Preferably, the iron oxide nanoparticles are coated with one polymeric compound or a mixture of polymeric compounds.

Specifically, the polymeric compound functionalizing the nanoparticles is chosen as a function of the amount of phosphate that needs to be extracted from the blood. Preferably, the dialysate composition according to the invention comprises at least one iron oxide nanoparticle functionalized with a mixture of polymeric compounds.

Typically, the polymeric compound can act as a surfactant.

The polymer may be chosen from anionic, cationic, nonionic, amphoteric and zwitterionic polymers and mixtures thereof.

Preferably, the polymeric compound is chosen from the group consisting of:
- polymers bearing catechol groups such as gallic acid, anionic polyphenol, tannic acid, dopamine and polydopamine;
- polymers bearing carboxylate groups such as polyacrylic acid, carboxymethyl dextran, hyaluronic acid, polyacrylates, polyethelylene glycol carboxylates;
- polysaccharides or polycarbohydrates;
- polyalcohol based polymers such as polyvinyl alcohol (PVA);
- cationic polymers such PolyEthylenelmine (PEI), PolyDiAllylDiMethylAmmonium Chloride (PDADMAC);
- biomolecules based such as peptide or proteins.

By "polysaccharide", it is meant a long-chain polymeric carbohydrate composed of monosaccharide units bound together by glycosidic linkages such as dextran, cellulose, amidon, hyaluronic acid and/or glucose.

Preferably, the polymers used for coating the nanoparticles are biocompatible and are not cytotoxic.

Preferably, the polymeric compound is chosen from anionic polymers and cationic polymers.

Preferably, the polymeric compound is chosen from tannic acid, polyacrylic acid, carboxymethyl dextran, hyaluronic acid and mixtures thereof.

According to another embodiment, the polymeric compound is a cationic polymer. According to this embodiment, the polymeric compound is chosen from polyethyleneimine (PEI), polydiallyldimethylammonium chloride (PDADMAC) and mixtures thereof.

Preferably, the iron oxide nanoparticle is coated with a polymeric compound selected from the group consisting of tannic acid, dextran, polyacrylic acid, PDAD, PEI and mixtures thereof.

According to an embodiment, the coated iron oxide nanoparticle is prepared by the polyol solvothermal method as described in :
Low Oxidation State and Enhanced Magnetic Properties Induced by Raspberry Shaped Nanostructures of Iron Oxide, O. Gerber, B. P. Pichon, C. Ulhaq, J.-M. Grenèche, C.Lefevre, I. Florea, O. Ersen, D. Begin, S. Lemonnier, E. Barraud, S. Begin-Colin, J. Phys. Chem. C 2015, 119 (43), 24665-24673 *; and*
*-* Synthesis Engineering of Iron Oxide Raspberry Shaped Nanostructures, O. Gerber, B. P. Pichon, D. Ihiawakrim, I. Florea, S. Moldovan, O. Ersen, D. Begin, J.-M. Grenèche, S.Lemonnier, E. Barraud, S. Begin-Colin, Nanoscale, 2017, 9, 305-313*.*

Typically this synthesis protocol has been modified to perform the synthesis in the presence of tannic acid and directly formed *in situ* tannic acid coated nanoparticles.

In brief, the polyol method involves suspending the metal precursor and polymeric compound, preferably iron chloride hexahydrate in a glycol solvent and subsequently heating the solution to a refluxing temperature. This technique has been used to synthesize metallic, oxide, and semiconductor NPs.

This method is further described in the experimental section.

Mono-metallic and metallic alloy NPs have been synthesized with this technique.

According to a second embodiment, the coated iron oxide nanoparticles is prepared by coprecipitation as described in:
- Daou, T. J.; Pourroy, G.; Bégin-Colin, S.; Grenèche, J. M.; Ulhaq-Bouillet, C.; Legaré, P.; Bernhardt, P.; Leuvrey, C.; Rogez, G. Hydrothermal Synthesis of Monodisperse Magnetite Nanoparticles. Chemistry of Materials 2006, 18 (18), 4399-4404*; and*
- Baaziz, W.; Pichon, B. P.; Fleutot, S.; Liu, Y.; Lefevre, C.; Greneche, J.-M.; Toumi, M.; Mhiri, T.; Begin-Colin, S. Magnetic Iron Oxide Nanoparticles: Reproducible Tuning of the Size and Nanosized-Dependent Composition, Defects, and Spin Canting. The Journal of Physical Chemistry C 2014, 118 (7), 3795-3810*.*

In brief, the co-precipitation method is a liquid phase synthesis method that involves mixing two solutions to obtain an insoluble compound (mixed oxides) by precipitation reaction. The polymeric compound could be introduced during or after the coprecipitation step and by using other bases such as ammoniac or NaOH.

This method is further described in the experimental section.

Preferably, this synthesis protocol has been modified to perform the synthesis in the presence of tannic acid and directly formed *in situ* tannic acid coated nanoparticles.

In a preferred embodiment, the iron oxide nanoparticle is coated with tannic acid.

Preferably, the iron oxide nanoparticle has a molar ratio polymer : iron (i.e. polymer: Fe) ranging from 0.001 to 0.05, preferably from 0.002 to 0.03 and more preferably from 0.005 to 0.02.

Preferably, when the polymer is PDADMAC, the iron oxide nanoparticle has a molar ratio polymer PDADMAC : iron ranging from 2.5 and 15, preferably from 10 and 15.

Preferably, the iron nanoparticle has an average hydrodynamic diameter ranging from 10 nm and 300 nm, preferably from 20 nm and 200 nm, and more preferably from 30 nm and 150 nm.

The hydrodynamic diameter is measured by dynamic light scattering (DLS) or transmission electron microscopy (TEM), which are methods well-known by the skilled person.

Note that the diameter that is measured in DLS is a value that refers to how a particle diffuses within a fluid so it is referred to as a hydrodynamic diameter. The diameter that is obtained by this technique is the diameter of a sphere that has the same translational diffusion coefficient as the particle.

The particle translational diffusion coefficient will depend not only on the size of the particle "core", but also on any surface structure that will affect the diffusion speed, as well as the concentration and type of ions in the medium. Factors that can affect the diffusion speed discussed in the following sections.

Preferably, the coated iron oxide nanoparticle has a specific surface area from 5 m²/g to 1000 m²/g, preferably from 10 m²/g to 500 m²/g and more preferably from 50 m²/g to 500 m²/g.

By "specific surface area" it is meant the total surface area of the coated iron oxide nanoparticle available for chemical or physical processes to take place. As physical or chemical processes, one can particularly cite absorption or adsorption.

The specific surface area can be determined by nitrogen adsorption-desorption isotherms using the BET method with Tristar equipment from Micromeritics. Prior to analysis, the sample was degassed under vacuum at 150 °C.

Preferably, the dialysate composition according to the invention has a concentration of coated iron oxide nanoparticles from 0.2 g.L⁻¹ to 500 g.L⁻¹, preferably from 0.2 gl.L⁻¹ to 5 g.L⁻¹.

Preferably, the dialysate composition according to the invention has a concentration of coated iron oxide nanoparticles from 0.04 mmol.L⁻¹ to 4 mol.L⁻¹, preferably from 0.04 mmol.L⁻¹ to 0.4 mol.L⁻¹, and even more preferably from 0.04 mmol.L⁻¹ to 20 mmol.L⁻¹.

Specifically, the concentration of coated iron oxide nanoparticles in the composition according to the invention can be adjusted according to the patient's needs.

Preferably, the dialysate composition according to the invention is a composition for hemodialysis.

Specifically, it is meant a composition meeting the requirements to be used for hemodialysis.

Hemodialysis is a process of purifying the blood of a person whose kidneys are not working normally. This type of dialysis achieves the extracorporeal removal of toxic products such as creatinine and urea and free water from the blood when the kidneys are in a state of kidney failure.

Precisely, hemodialysis utilizes counter current flow, where the dialysate is flowing in the opposite direction to blood flow in the extracorporeal circuit. Counter-current flow maintains the concentration gradient across the membrane at a maximum and increases the efficiency of the dialysis. Fluid removal is achieved by altering the hydrostatic pressure of the dialysate compartment, causing free water and some dissolved solutes to move across the membrane along a created pressure gradient.

Preferably, the dialysate composition according to the invention is for hemodialysis, and comprises:
(a) at least one pH buffer,
(b) at least one mineral salt, and
(c) at least one iron oxide nanoparticle (Fe²⁺ and/or Fe³⁺) coated with at least one polymeric compound.

According to another embodiment, the dialysate composition according to the invention is a composition for peritoneal dialysis.

Specifically, it is meant a composition meeting the requirements to be used for peritoneal dialysis.

Peritoneal dialysis is a way to remove toxic products from the blood. During peritoneal dialysis, a cleansing fluid flows through a tube into part of the stomach area, also called the abdomen. The inner lining of the abdomen, known as the peritoneum, acts as a filter and removes toxic products from blood. After a set amount of time, the fluid with the filtered toxic flows out of the abdomen and is thrown away. By diffusion and convection (pressure gradients) mechanisms through the capillaries, toxins and water in excess pass into the dialysate. The dialysate is then drained outside the body before the subsequent provision of new dialysis fluid. The duration of the exchanges varies according to the needs of the patient and the chosen technique.

Peritoneal dialysis differs from a more-common procedure as it works inside the body to clean the blood.

However, peritoneal dialysis requires that iron oxide nanoparticles (IONPs) do not cross the peritoneal membrane. This membrane surrounds all the organs of the abdominal cavity, delimiting a peritoneal cavity.

The peritoneal membrane is composed by a layer of flattened cells and an underlying area of varying thickness (the interstitium) containing lymphatic vessels, blood capillaries, and nerves. The exchange between the blood and the peritoneal cavity needs to cross the endothelium of the capillaries, flattened cells (mesothelial cells), and the space between both. The mechanism of passage through the membrane is described by the three-pores model.

This model of peritoneal transport is used extensively for modeling peritoneal fluid and solute transport. This model characterized the passage through the membrane through three various sized pores.
i) Ultra-small pores with a radius of 2 to 4 Å. These are the most numerous and correspond to aquaporins. They only allow the passage of water by osmosis, through the cells.
ii) Small pores with a radius of 40 to 55 Å, allowing water and solutes of low to medium molecular weight to pass through convection (osmotic and hydrostatic pressure gradient) and diffusion (concentration gradient) processes.
iii) Large pores with radius of 250 to 300 Å allowing the transport of large molecules by convection (hydrostatic pressure gradient) and diffusion processes.

Therefore, to avoid transfer of IONPs from dialysis solution to blood vessels, they need a large mean diameter.

Preferably, the composition according to the invention is for peritoneal dialysis, and comprises:
(a) at least one pH buffer,
(b) at least one mineral salt,
(c) at least one osmotic agent, preferably selected form the group consisting of glucose, icodextrin and mixtures thereof; and
(d) at least one iron oxide nanoparticle (Fe²⁺ and/or Fe³⁺) coated with at least one polymeric compound.

The composition according to the invention may further comprise another nanoparticle selected from the group consisting of inorganic nanoparticles, polymeric nanoparticles, polymeric capsules, organic-inorganic hybrid nanoparticles and organic-inorganic hybrid capsules.

Preferably, the dialysate composition may further comprise one or more active ingredient chosen from anti-cancer agents, analgesic agents, antibacterial agents, antibiotics, antiseptics, antivirals agents and hormones.

The present invention also relates to the use of the dialysate composition as described above as a medicament.

The composition according to the invention may be used as a dialysate for biological fluid and/or blood purification.

By "blood purification", it is meant removing toxic products from the blood such as toxins and phosphate that are normally regularly eliminated by healthy kidneys.

After blood purification, the range of said toxic product(s) is(are) typically reduced to a level at which it (they) is(are) no longer toxic to the human body.

Preferably, the range of phosphate in blood is comprised between0.85 mg/L and 1.5 mg/L. Preferably, the range of phosphate in children's blood is less than 2.5 mg/L.

Preferably, the range of phosphate removed by the dialysate composition after hemodialysis or peritoneal dialysis is more than 50%, preferably 60% and even more preferably 70% of the initial phosphate range in the human body.

Typically, hemodialysis session lasts from 3 to 5 hours, during which the patient is connected to the device and cannot move. At least, 3 sessions per week are necessary to allow an effective purification. Phosphate and toxins removal is more efficient by HD than by PD. PD session is usually performed over night when using a cycler (automated peritoneal dialysis). In case of continuous ambulatory peritoneal dialysis, the patient does not need to be branched to a machine, he can process to the infusion and then disconnects the system and therefore has movement liberty, the possibility of being autonomous in the treatment. PD has numerous advantages: fewer hospitalizations are needed, no anticoagulants are necessary and therefore, the patients have less side effects and there is the possibility to perform the treatment during the night/sleep with a cycler. It allows for less diet and fluid restriction for the patient and a better vascular preservation compared to HD. Finally, this method is the sole dialysis process especially for infants weighing less than 6-8 kg and new -born babies where intermittent chronic HD is difficult to perform due to their too low blood volume.

By "toxic products" it is meant products from the blood which need to be removed. By biological fluids or toxic blood products, it is generally mentioned phosphate or uremic toxins.

In parallel, the accumulation of toxins has also negative effects on physiological functions, resulting in a gradual endogenous intoxication and in a progressive deterioration of the clinical conditions of the patients.

Uremic toxins are classified in three groups associated with their molecular weight and chemical features:
- free water-soluble compounds (WS) with a molecular weight less than 500 Dalton, such as creatinine and urea;
- middle molecular weight compounds (MM) with a molecular weight comprised between 500 to 60 000 Da. These compounds are typically peptides and proteins; such as β2-microglobulin; and
- protein-bound uremic toxins (PBUTs), which are relatively low molecular weight molecules that bind strongly to proteins in the blood, such as albumin. The main molecules in this group, which have a higher than 90% protein bound percentage, are indoxyl sulfate (IS) and p-cresyl sulfate (PCS).

It is also described a method of treatment to purify a patient's blood or a biological fluid, preferably to remove phosphate and at least one toxin, comprising the following steps:
- providing a composition as described above; and
- administering the composition according to the invention to the patient via a device designed for haemodialysis.

It is also described a method of treatment to purify a patient's blood, preferably to remove phosphate, comprising the following steps:
- providing a composition as described above; and
- administering the composition according to the invention to the patient via a device designed for peritoneal dialysis.

By "patient" it is meant a human with kidney function problems, preferably a human having chronic kidney disease.

The present invention is now illustrated by the following figures and examples.

### Figures

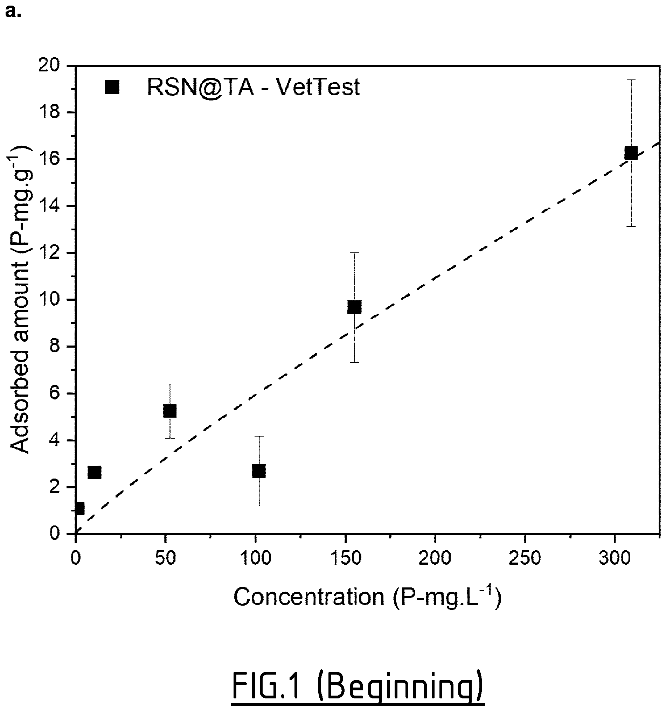

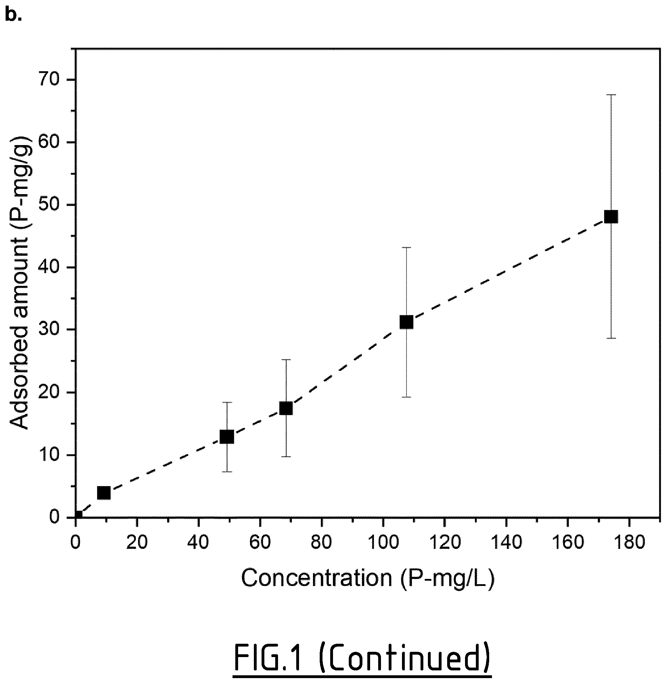

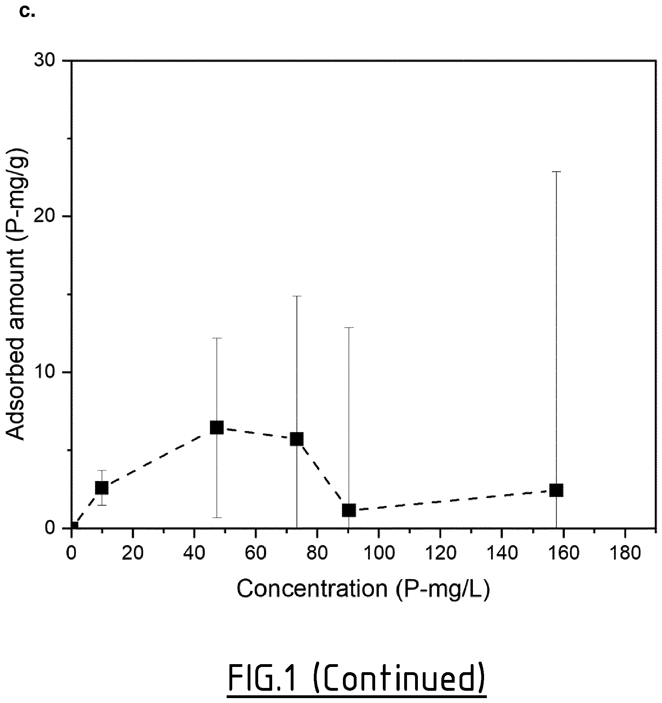

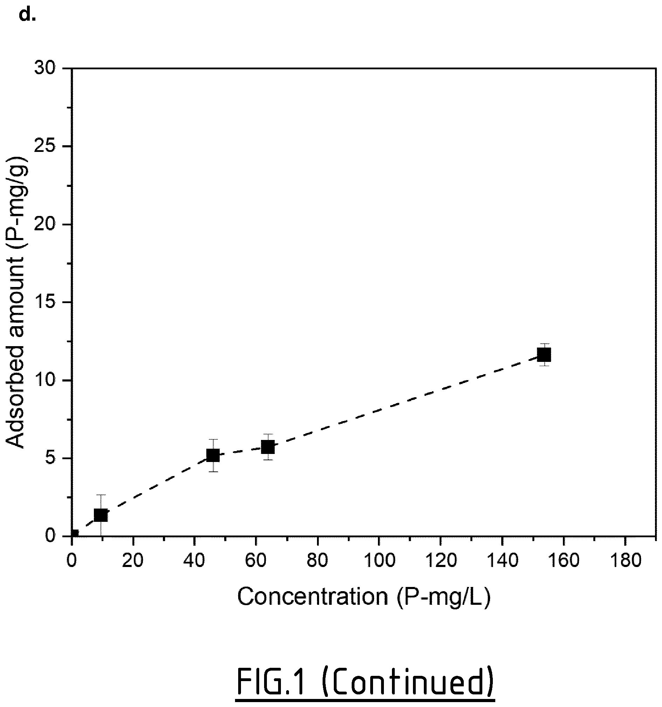

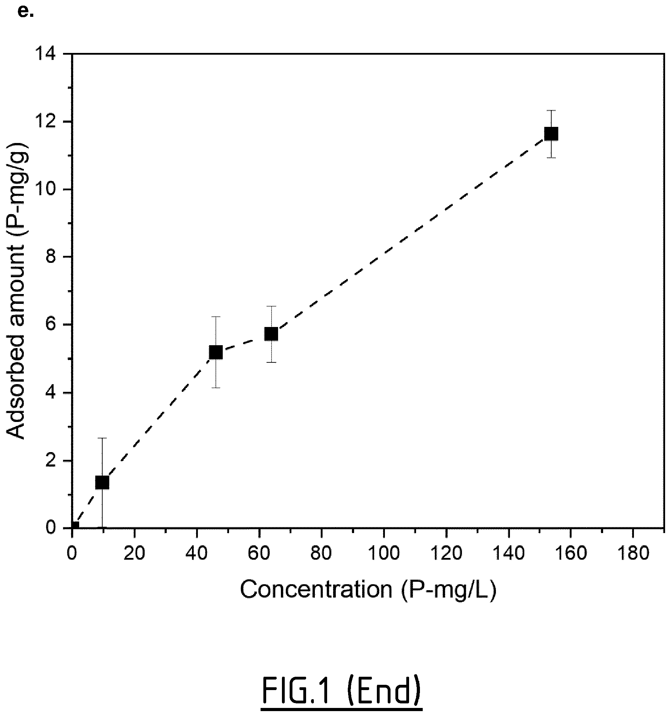

**Figure 1****:** Phosphate isotherm adsorption experiment on the surface of (a) raspberry-shaped nanostructures obtained by polyol solvothermal method coated with the anionic polyphenol tannic acid, (b) iron oxide nanoparticles obtained by coprecipitation method coated with the cationic polymer PDADMAC, (c) iron oxide nanoparticles obtained by coprecipitation method coated with the anionic polymer PAA and (d) iron oxide nanoparticles obtained by coprecipitation method coated with tannic acid and (e) iron oxide nanoparticles obtained by coprecipitation method coated with the anionic polymer PAA.
**Figure 2****:** Phosphate kinetic adsorption experiment on the surface of Raspberry-Shaped Nanostructures obtained by polyol solvothermal method without or with coating of tannic acid (a) at pH 3, (b) at pH 7 and (c) at pH 7 quantified by VetTest and molylbdenum blue methods.
**Figure 3****:** Isotherm adsorption experiments of uremic toxins - (a) creatinine, (b) urea, (c) indoxyl sulfate and (d) p-cresol - and beneficial compounds - (e) glucose and (f) calcium - on the surface of Raspberry-Shaped Nanostructures obtained by polyol solvothermal method, without or with coating of tannic acid.
**Figure 4****:** Diffusion rate over time of methylthioninium chloride (MB), fluorophore-labeled glucose (marked glucose) and fluorescein isothiocyanate labelled bovine serum albumin (BSA-FITC) in cassette mode experiments through a 20 kDa membrane made of regenerated cellulose.
**Figure 5****:** Phosphate diffusion over time at room temperature for different dialysis and dialysate compositions in (a) tubing mode experiment and (b) cassette mode experiment.
**Figure 6****:** Phosphate diffusion rate over time in cassette mode experiment with different parameters mimicking physiological conditions.
**Figure 7:** (a) Phosphate diffusion rate over time in tubing mode experiment in the presence of iron oxide nanostructures coated with different surfactant in the dialysate compartment and (b) additional quantity of phosphate diffused per mass unit of material at 4 h in comparison with reference experiment.
**Figure 8:** Phosphate diffusion rate over time in cassette mode experiment in the presence of iron oxide nanostructures coated with different surfactant in the dialysate compartment.
**Figure 9****:** Phosphate diffusion rate over time in cassette mode experiment for different dialysate solution composition, performed at room temperature, in the presence of Iron Oxide Nanoparticles obtained by coprecipitation method, coated with tannic acid in the dialysate compartment.

### Examples

### Example 1: Preparation of iron oxide nanoparticles coated with tannic acid by a polyol solvothermal method

### 1.1. Polyol solvothermal synthesis conditions of nanoparticles without and with surfactants

The iron oxide raspberry-shaped nanoclusters without and with surfactant are prepared by a polyol solvothermal method, following a previously established method¹. 1.626 g of FeCl3.6H2O, 0.24 g of succinic acid and 3.6 g of urea are added to 60 mL of ethylene glycol and placed under magnetic stirring to ensure the dissolution of all reagents before the addition of surfactant.

During synthesis in the presence of tannic acid, quantities of surfactant are added in the reaction mixture such as molar ratio TA:Fe = 0.001, 0.005, 0.075, 0.01, 0.015 or 0.02. During synthesis in the presence of polyacrylic acid, 1 mL of ultrapure water is added to the reaction mixture followed by quantities of polyacrylic acid 1800 Mw such as molar ratio PAA:Fe = 1.33 or 2.0.

During synthesis in the presence of positive surfactants such as polyethylenimine (50% mass in water 60,000 Mn), quantities of surfactant is added in the reaction mixture such as mass ratio PEI:Fe = 1.5.

The mixture is then placed again under magnetic stirring for 2 h and, followed by 1 h of ultrasounds. The reaction mixture is then sealed in a stainless-steel autoclave lined with an inner Teflon reactor of 75 mL and placed inside an oven. The reaction mixture is slowly heated to 200 °C at 1.5 °C/min and left at 200°C for 10.5 h before being cooled to room temperature outside the oven. The synthesised materials are then separated magnetically and washed 9 times with a mixture of 50% ethanol/50 % acetone (4 min under ultrasounds). During synthesis with the surfactant polyacrylic acid and polyethylenimine, the synthesised materials are separated magnetically and washed 9 times with a mixture of 50% ethanol/50 % distilled water (4 min under ultrasounds).

During synthesis where non-magnetic materials are obtained, centrifugation are used to wash the materials.

After washing, the synthesized nanoclusters are stored in ethanol until further characterization or use, or distilled water for immediate use.
[1] Gerber, O.; Pichon, B. P.; Ihiawakrim, D.; Florea, I.; Moldovan, S.; Ersen, O.; Begin, D.; Grenèche, J.-M. .; Lemonnier, S.; Barraud, E.; Begin-Colin, S. Synthesis Engineering of Iron Oxide Raspberry-Shaped Nanostructures. Nanoscale 2017, 9 (1), 305-313

### 1.2. Preparation of iron oxide nanoparticles coated with tannic acid and polyacrilic acid by coprecipitation and with introduction of the polymeric compound during the synthesis

The iron oxide nanoaggregates without and with surfactant are prepared by a coprecipitation method, following a previously established method¹ by introducing the surfactant either during or after the coprecipitation step and by using other bases such as ammoniac or NaOH..

During a synthesis without surfactant, at first, 2.7 g of FeCl3.6H2O and 1 g of FeCl2.6H2O are added to 12.5 mL of hydrochloric acid 2M solution and placed under magnetic stirring to ensure the dissolution of all reagents. The reaction mixture is then transferred in a tri-col balloon and bubbled under nitrogen atmosphere during 15 min.

During synthesis in the presence of **tannic acid**, 4.1 g FeCl3.6H2O only is added to 12.5 mL of hydrochloric acid 2M solution with a quantity of tannic acid such as molar ratio TA:Fe = 0.01..

During a synthesis in the presence of **polyacrylic acid**, a quantity such as the molar ratio PAA/Fe = 0.2 or 0.4 is quickly added to the reaction mixture after the degassing step.

### Polycation: the polycations are added with the base

Still under nitrogen atmosphere, the balloon is placed in an oil bath and heated to 70°C at approximatively 1.7°C/min while the reaction mixture is stirred with a stirring blade connected to a motor. A given amount of base (for example: 26 mL of tetramethylammonium hydroxide 25%) is then added drop by drop with a peristaltic pump at a flow rate of 0.7 mL/min. At the end of the injection, the balloon is removed from the oil bath and let to cool to room temperature. The synthesised materials are centrifugated and washed 4 times with previously deoxygenated distilled water to prevent material oxidation. After washing, the synthesized material is stored in deoxygenated water at 4°C until further characterization or use.
[1] Daou, T. J.; Pourroy, G.; Bégin-Colin, S.; Grenèche, J. M.; Ulhaq-Bouillet, C.; Legaré, P.; Bernhardt, P.; Leuvrey, C.; Rogez, G. Hydrothermal Synthesis of Monodisperse Magnetite Nanoparticles. Chemistry of Materials 2006, 18 (18), 4399-4404.

### 1.3. Preparation of iron oxide nanoparticles coated with tannic acid and poly(diallyldimethylammonium chloride by coprecipitation and with introduction of the polymeric compound after the synthesis

During a coating process with the anionic surfactants (tannic acid and polyacrylic acid), suspensions of 50 mL of iron oxide nanoparticles at concentration 0.1 or 0.5 mg(Fe3-xO4)/mL are prepared with ultrapure water without modification of pH (typically, pH = 9 after washings during coprecipitation method) and placed in an ultrasound bath for ensuring deagglomeration of nanoaggregates. Still under ultrasound, pH is quickly and carefully adjusted at 5 with hydrochloric acid solution to avoid the isoelectric point of iron oxide. The suspension is placed under ultrasound for 30 additional minutes, followed by the introduction of an amount of tannic acid or polyacrylic acid 1800 Mw such as molar ratio TA:Fe or PAA:Fe = 0.01. The suspension is then placed on a mechanical stirrer for 12 h. After the coating step, the suspension is washed 9 times by ultrafiltration with ultrapure water and the pH is carefully adjusted to 7 with hydrochloric acid or sodium hydroxide solution if needed. The material is then stored in ultrapure water at 4°C until further characterization or use.

During a coating process in the presence of cationic surfactants such as poly(diallyldimethylammonium chloride) and PEI and PAH, suspension of 10 mL of iron oxide nanoaggregates at concentration 3.0 mg(Fe3-xO4)/mL are prepared with ultrapure water without modification of pH (typically, pH = 9 after washings during coprecipitation method). Then, 40 mL of surfactant such as poly(diallyldimethylammonium chloride) solution at concentration 10 mg/mL is slowly added to the initial suspension. Among the experiments conducted, the mass ratio of poly(diallyldimethylammonium chloride):Fe was kept above 10. The suspension is then stirred on a mechanical stirrer for 12 h. The material is centrifugated and washed 3 times with ultrapure water before being suspended in ultrapure water and stored at 4°C until further characterization or use.

### 1.4. Preparation of iron oxide nanoparticles coated with tannic acid by thermal decomposition and with introduction of the polymeric compound after the synthesis

The 10 nm sized iron oxide nanoparticles are prepared by a thermal decomposition method, following a previously established method¹. In a standard synthesis 1.83 g of iron(III) stearate and 1.24 g of oleic acid are added to 20 mL of dioctyl ether in a balloon flask and placed on a heater connected to a computer controlling the temperature rise using a system of thermocouples. The reaction mixture is placed under magnetic stirring while being heated at 100°C for 1 h. After retrieving the magnetic stirrer and connecting a cooler, the reaction mixture is heated from 100°C to 292°C at 5°C/min then held at 292°C for 2 h. The reaction mixture is then let to cool to room temperature. The synthesised materials are separated magnetically and washed 3 times in 400 mL of warm acetone stirred with a stirring blade connected to a motor. After washing, the synthesized material is stored in tetrahydrofuran until further characterization or use, or chloroform for immediate use.

For the synthsesis of 5 nm sized nanoparticles, the solvent was hexadecene and the iron precursor was iron II stearate.

During a coating process with the surfactant tannic acid, suspensions of 47 mL of iron oxide nanoparticles at concentration 0.1 or 0.2 mg(Fe3O4)/mL are prepared in tetrahydrofuran and placed in an ultrasound bath for ensuring deagglomeration of nanoaggregates. A solution of 3 mL of tannic acid in dimethylformamide at concentration 0.0025 mmol is then added to the nanoparticles suspension followed by 0.25 mmol of a solution of 2-(2-aminoethoxy)ethanol. The mixture is placed under magnetic stirring in an oil bath and the temperature adjusted to 50°C for 18 h. After the functionalization step, the suspension is precipited in diethyl ether and centrifuged. The precipitate is then washed and centrifuged with tetrahydrofuran to remove free molecules of octyl ether. The material is then suspended in distilled water until further characterization or use.

### 1.5. Elaboration of capsules containing iron oxide nanoparticles

This approach involves the sequential adsorption of alternating layers of oppositely charged polymers (polycations and polyanions) on the surface of nanoparticles. The adsorption is driven by electrostatic interactions between the layers of polymers. Parameters like the thickness, the composition and the properties of the surface can be modulated based on the choice of the polymers and on the amount of layers deposited.
Polyanion: Tannic Acid crosslinked (TA*)
Polycation: poly-(diallyl dimethylammonium chloride) (PDAD)
Nanoparticles: Iron Oxide Nanoparticles with a shell of mesoporous silica IONPs@mSiO₂ (NP)

100mg of IONPs@mSiO₂were first mixed with 25 mL of 10mg/mL PDAD solution and sonicated for 10'. The mixture was then stirred for 80' at room temperature (RT) with three subsequent washes using water. Next, 25 mL of 2 mg/mL of TA solution were added to NP@PDAD and sonicated for 10'. The resulting suspension was again stirred for 80' minutes at RT, followed by three washes with water. For the cross-linking (*) of TA, 5 mL of NP@PDAD@TA suspension were mixed with 10 mL of Phosphate buffer (pH 7) and 10 µL of 40 wt% FeCl₃.6H₂O. The mixture was left for complexation for 6 hours, followed by three washes with water. Subsequently, the same protocol was repeated to add in total, 4 layers of polymers on the top of the NP.

### Example 2: Characterization of the iron oxide nanoparticles with tannic acid

### 2.1. Materials and methods

Fourier Transform Infrared Spectroscopy (FTIR) was used to identify the chemical groups present in RSN and RSN-TA. The IR spectra were recorded in a Perkin Elmer Spectrum 100 spectrometer for wavenumbers between 4000 and 400 cm⁻¹. For the preparation of the samples, a few drops of the suspension of composites in ethanol were mixed with dry KBr and after the evaporation of the solvent, pellets were prepared and FTIR transmittance spectra were obtained.

X-Ray Diffraction (XRD) was used to identify the crystalline phases present. The X-Ray Diffractometer was a Bruker D8 Discover, equipped with a Lynx-Eye detector in the 20-70° (2θ) range with a scan step of 0.03° and the sample rotates at 30 rpm during the measure. Silicon powder was used as internal standard. The diffraction peaks were compared to JCPDS database. Profile matching with the software Fullprof was used to obtain estimates of the crystallite size and lattice parameter.

Scanning Electronic Microscopy (SEM) was used to obtained images of the synthesized nanoclusters, using a Zeiss Gemini SEM 500 electronic microscope operating at 3.00 kV. The materials were deposited in silicon wafers and posteriorly observed by SEM.

Transmission Electronic Microscopy (TEM) was performed to obtain images of the synthesized materials. For this, a high-resolution transmission electron microscope Topcon 002B operating at 200 kV was used. The samples were deposited on carbon-coated copper grids. After the image acquisition, the software Image J was used to obtain the size distribution of the iron oxide nanostructures.

Thermal Gravimetric Analysis (TGA) was performed on a SDT 600 from TA Instruments to measure the loss mass of the sample when the temperature changed and quantify the amount of tannic acid present in the nanoclusters. The measurements were performed under air flow, from room temperature until 900°C with a heating rate of 5°C/min. The materials were previously dried, before analysis.

Dynamic Light Scattering. Dynamic Light Scattering measurements were performed on a Zetasizer Nano ZS from Malvern Panalytical to assess the colloidal stability of the RSN and RSN-TA suspensions in water or in dialysates and to determine their mean hydrodynamic diameter (Dh). The electrophoretic mobility was also recorded using the same equipment in order to obtain the zeta potential value at the pH of the solution and to determine the isoelectric point of the nanoparticles. The zeta potential measurements were performed at 25°C. The composites were suspended in water and sonicated for 10 minutes prior to analysis. The pH was adjusted with HCl and NaOH 0.1 M solutions.

### 2.2. Characterization of nanomaterials

The characteristics of the different nanoclusters deduced from, DLS, IR SEM and TEM images are also summarized in Table 1.

**Table 1. Summary of the microstructural characteristics of samples**

| **Name of nano-materials** | **Synthesis method** | **Name of surfactant** | **Introduction of surfactant** | **Modified parameter / standard synthesis** | **NP size by TEM (nm)** | **Nano-cluster/ aggregate size by TEM (nm)** | **Mean hydro-dynamic size at pH 7 (nm)** | **Zeta potential value (mV)** | **IR spectro scopy** |
|---|---|---|---|---|---|---|---|---|---|
| RSN@TA | Polyol solvothermal | TA | During synthesis | 0% H20 in reaction mixture | 9±3 | 264±60 | 368±14 | -28±1 | IR bands of TA and iron oxide |
| RSN@TA | Polyol solvothermal | TA | During synthesis | 5% H2O in reaction mixture | 9±3 | 257±61 | 575±8 | -41±1 | IR bands of TA and iron oxide |
| RSN@TA | Polyol solvothermal | TA | During synthesis | 8% H2O in reaction mixture | 9±3 | 242±42 | 547±21 | -38±1 | IR bands of TA and iron oxide |
| RSN@TA | Polyol solvothermal | TA | During synthesis | 16% H2O in reaction mixture | 9±3 | / (No specific aggregatio n shape) | 740±20 | -33±1 | IR bands of TA and iron oxide |
| RSN@TA | Polyol solvothermal | TA | During synthesis | Urea (basic agent) and succinic acid replaced by sodium acetate (basic agent) and PEG 6000 (stabilizer) | 3±2 | 151±32 | 320±22 | -43±1 | IR bands of TA and iron oxide |
| RSN@TA | Polyol solvothermal | TA | During synthesis | X2 quantity of reagents in reaction mixture | 4±2 | 188±40 | / | / | / |
| RSN@TA | Polyol solvothermal | TA | During synthesis | /2 quantity of reagents in reaction mixture | 5±2 | 329±33 | 783±10 | -46±1 | / |
| RSN@PA A | Polyol solvothermal | PAA | During synthesis | Surfactant = PAA with molar ratio PAA:Fe = 1.3 | 5±3 | 322±53 | / | / | IR bands of PAA and iron oxide |
| RSN@PA A | Polyol solvothermal | PAA | During synthesis | Surfactant = PAA with molar ratio PAA:Fe = 2 | 5±3 | 336±53 | 650±85 | -38±1 | IR bands of PAA and iron oxide |
| RSN@PEI | Polyol solvothermal | PEI | During synthesis | Surfactant = PAA with molar ratio PEI:Fe = 13 | No statisti cal counti ng | No specific aggregatio n shape | / | / | / |
| IO-CP@TA | Coprecipitati on | TA | During synthesis | Addition of TA in initial acidic reaction mixture, magnetic stirring for 1 h, molar ratio TA:Fe = 0.01 | 9±3 | 27 | 110±1 | -40±1 | IR bands of TA and iron oxide |
| IO-CP+TA | Coprecipitati on | TA | Post synthesis | Addition of TA in acidic pH-adjusted suspension of material, molar ratio TA:Fe = 0.01 | 9±3 | / | 226 | -34 | IR bands of TA and iron oxide |
| IO-CP@PAA | Coprecipitati on | PAA | During synthesis | Addition of PAA in degassed acidic reaction mixture, molar ratio PAA:Fe = 0.4 | 7±1 | 70 | 136 | -43 | IR bands of PAA and iron oxide |
| IO-CP@PAA | Coprecipitati on | PAA | During synthesis | Addition of PAA in degassed acidic reaction mixture, molar ratio PAA:Fe = 0.2 | 6±1 | 30 | 71 | -49 | IR bands of PAA and iron oxide |
| IO-CP+PAA | Coprecipitati on | PAA | Post synthesis | Addition of PAA in acidic pH-adjusted suspension of material | 9±3 | / | 310 | -52 | IR bands of PAA and iron oxide |
| IO-CP+PDAD MAC | Coprecipitati on | PDADMA C | Post synthesis | Addition of PAA in basic pH suspension of material | 9±3 | / | 207 | 13 | / |
| IO-CP+PEI | Coprecipitati on | PEI | Post synthesis | Addition of PAA in basic pH suspension of material | 9±3 | / | 218 | 7 | / |
| IO-TD+TA | Thermal decompositi on | TA | Post synthesis | Addition of TA in a suspension of material in organic solvent with molar ratio TA:Fe = 0.01 | 15±3 | 41±32 | 180±1 | -36±1 | IR bands of TA and iron oxide |

### 2.3. Impact of the ratio of tannic acid on the microstructural characteristics of the iron oxide nanoparticles

The characteristics of the different nanoclusters deduced from SEM and TEM images are also summarized in Table 2, including the results obtained with higher ratios of TA:Fe..

The ratio 0.01 was found to be the optimal ratio to have a low mean hydrodynamic size and a high yield in RSN TA (quite no iron carbonates).

**Table 2. Summary of the microstructural characteristics of samples obtained with different TA:Fe ratios**

| **Molar Ratio TA:Fe** | **0** | **0.001** | **0.005** | **0.007** | **0.010** | **0.015** | **0.020** |
|---|---|---|---|---|---|---|---|
| **NC particle size distribution (nm)*** | 312 ± 126, 391 ± 60 | 278 ± 137, 352 ± 49 | 190 ± 74, 327 ± 107, 453 ± 67 | 139 ± 60, 330 ± 193, 421 ± 36 | 173 ± 30, 292 ± 98, 361 ± 50 | - | - |
| **NC grain size (nm)*** | 44.5 ± 22.2 | 20.6±9.0 | 10.4 ± 6.6 | 7.8 ± 2.8 | 6.4 ± 2.7 | - | - |
| **NC crystallite size (nm)**** | 38.4 | 7.4 | 10.8 | 9.4 | 6.5 | **-** | **-** |
| **NC lattice parameter (nm)**** | 8.406 | 8.413 | 8.402 | 8.401 | 8.409 | - | - |
| **FeCO₃ detected (XRD)** | No | No | No | No | Yes | Yes | Yes |
| **FeCO₃ detected (SEM) ^{a}** | No | No | Yes ★★ | Yes ★★ | Yes ★★★ | Yes ★★★★★ | Yes ★★★★★ |
| **Weight loss in TGA(%)** | 3.0 | - | 7.6 | 13.4 | 17.7 | - | 46.2 |

| | | | | | | | |
|---|---|---|---|---|---|---|---|
| ^{a} stars used to indicate, qualitatively, the amount of FeCO₃ detected: ★ - very low amount, ★★- low amount, ★★★ - medium amount, ★★★★ - high amount, ★★★★★ - very high amount. ^{∗} determined from SEM images, ^{∗∗} determined from XRD pattern. | | | | | | | |

### 2.4. Characterization of the micro-capsules

The hydrodynamic size and zeta potential were assessed during the preparation of the microcapsule. The results are summarized in table 3.

| | **NP** | **NP@PD AD** | **NP@PDAD @TA*** | **NP@PDAD@TA* @PDAD** | **NP@PDAD@TA*@ PDAD@TA*** |
|---|---|---|---|---|---|
| **Hydrodynamic Size (nm)** | 220 | 350 | 420 | 452 | 509 |
| **Zeta potential (mV)** | -30 | + 48 | -28 | + 27 | -28 |

| | | | | | |
|---|---|---|---|---|---|
| **Table 3.** Summary of the hydrodynamic size and zeta potential during the preparation of the micro capsules | | | | | |

### Example 3: Adsorption studies of phosphate, uremic toxins and compounds on nanomaterials

### 3.1. Phosphate isotherm adsorption on materials

Phosphate adsorption isotherm on nanomaterials were performed. A fixed amount of nanomaterial of 1000 mg.mL⁻¹ suspended in 20 mL solution of ultrapure water was putted in contact with compounds at different concentrations. All experiments were performed at room temperature, with a contact duration of 4 h. The amount of phosphate adsorbed were assessed for five different nanomaterials synthesized by various methods and coated with different surfactant.

Figure 1. a-e show results for five different nanomaterials synthesized by various methods and coated with different surfactant. Adsorbed amount of phosphate on each material at phosphate concentration of 50 P-mg.g⁻¹ are presented in table 4.

| **Material** | **Phosphate adsorbed amount (P-mg.g⁻¹)** |
|---|---|
| RSN@TA | 4.3 |
| IO-CP+PDADMAC | 12.9 |
| IO-CP@TA | 6.4 |
| IO-CP@PAA | 5.2 |

| | |
|---|---|
| **Table 4.** Amount of phosphates adsorbed depending on the nature of the nanoparticle | |

Materials coated with anionic polymers showed a similar performance in phosphate adsorption (Figures 1 and table 4.). IONPs coated with cationic polymer possesses the highest adsorption capacity due to electrostatically favorable interactions between positively charged polymer and negatively charged phosphate anions at pH 7.

### 3.2. Phosphate kinetic adsorption on materials

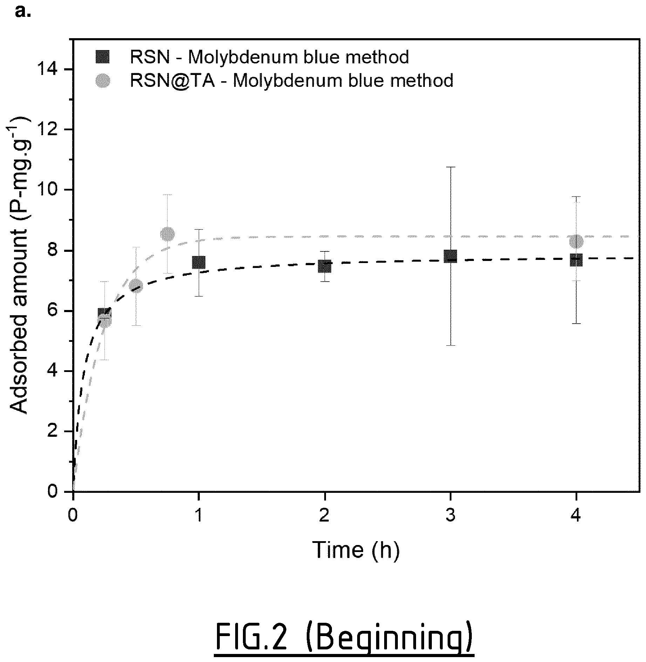

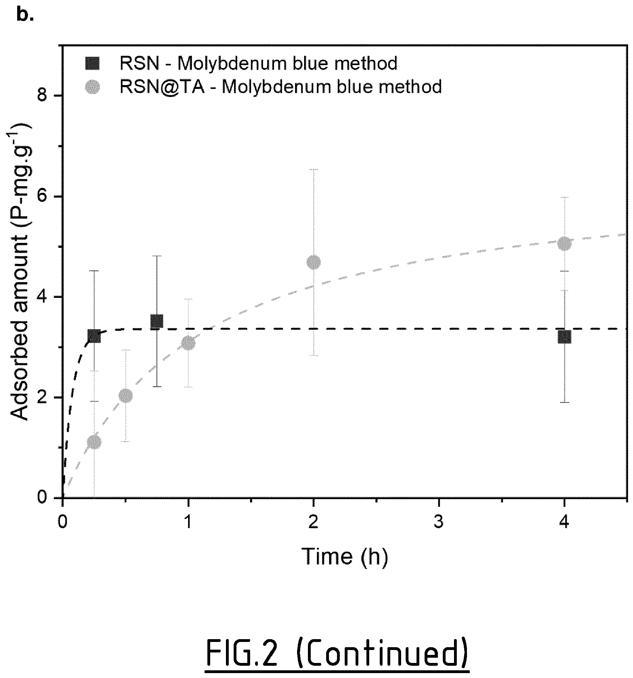

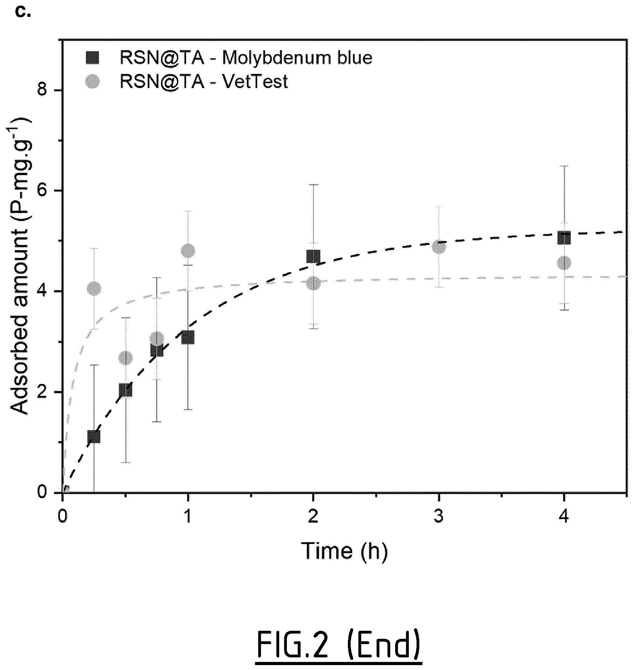

Phosphate kinetic adsorption experiments were performed at pH 3 and pH 7 (Figure 2). It has been confirmed that both nanomaterials were able to adsorb a significant amount of phosphate at acidic pH and at physiological pH (7.9 P-mg.g⁻¹ and 8.5 P-mg.g⁻¹ phosphate adsorbed respectively on RSN and RSN@TA at pH 3 and 5.2 and 3.2 P-mg.g⁻¹ phosphate adsorbed respectively on RSN and RSN@TA at pH 7). A higher amount of phosphate was adsorbed at pH 3 due to optimized electrostatic interaction between iron oxide surface and phosphate anions. At each pH value, RSN@TA showed a higher phosphate adsorption capacity than naked RSNs. All experiments indicate that maximum phosphate adsorption occurred from 2 h. Finally, figure 2.c show experiments that allowed to compare VetTest and molybdenum blue methods in quantification of phosphates and the results were found very similar. However, molybdenum blue method showed a higher standard deviation than use of VetTest, confirming this last method could be favorized for phosphate quantification.

### 3.3. Isotherm adsorption of uremic toxins and beneficial compounds on materials

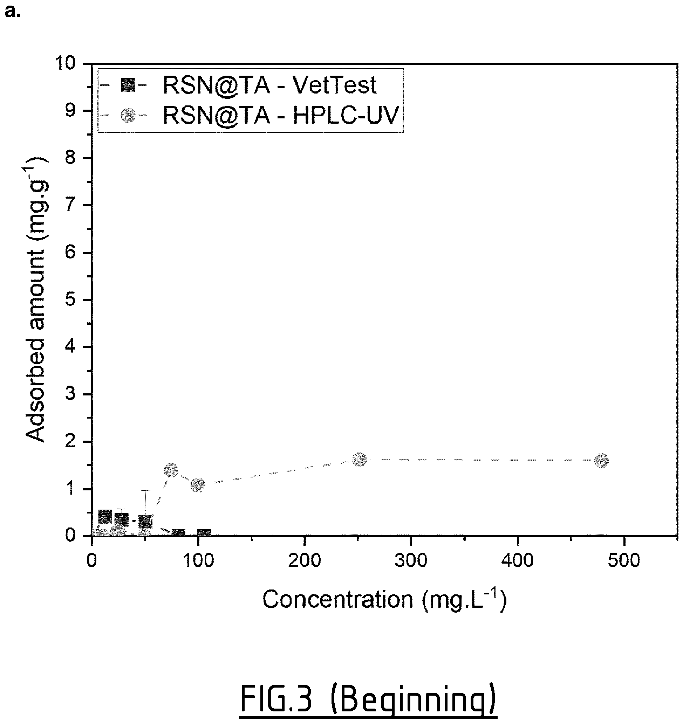

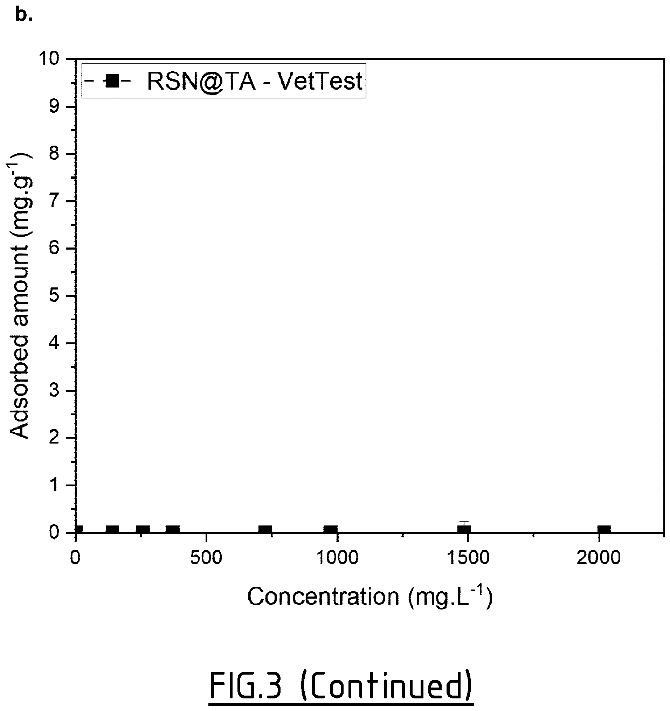

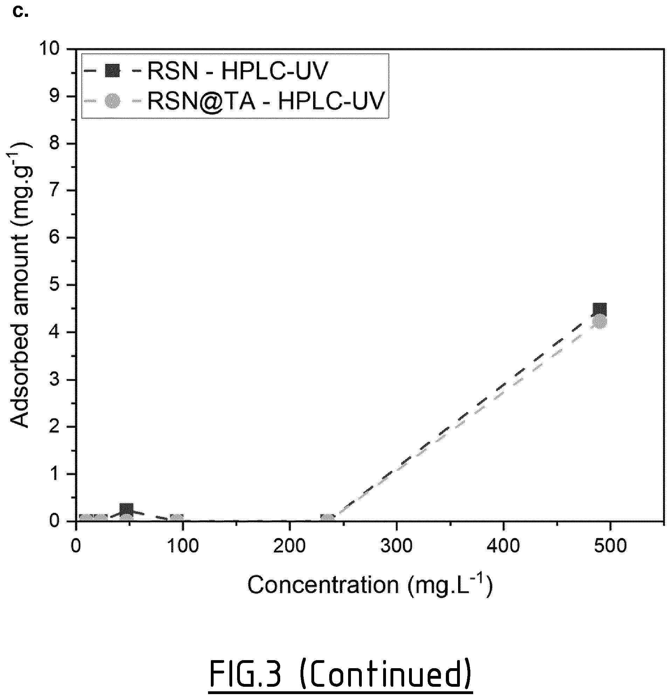

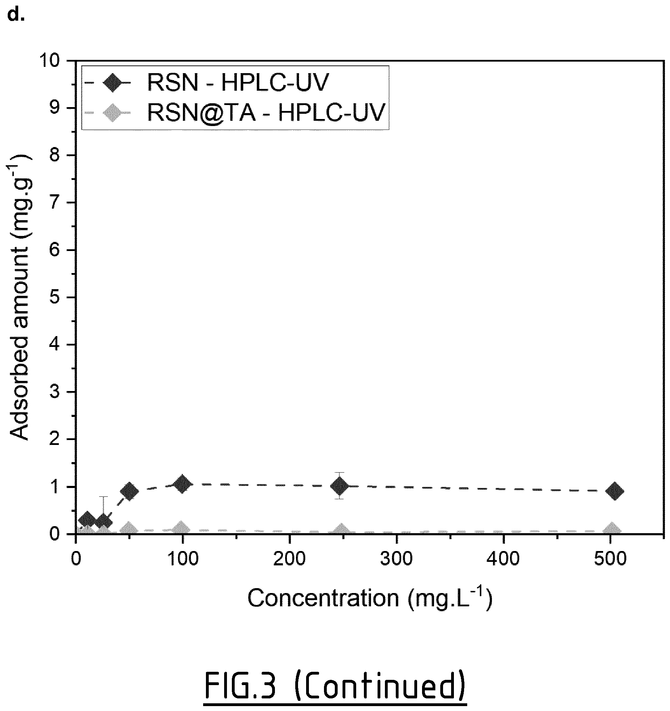

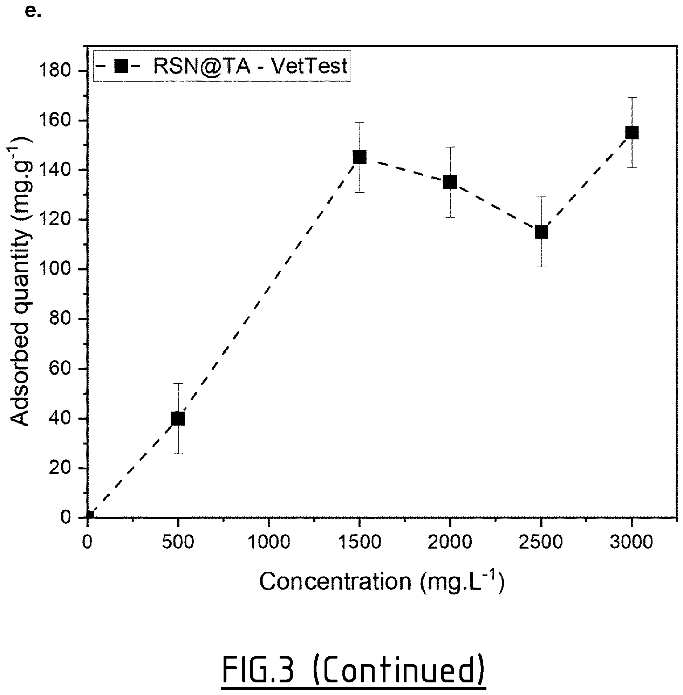

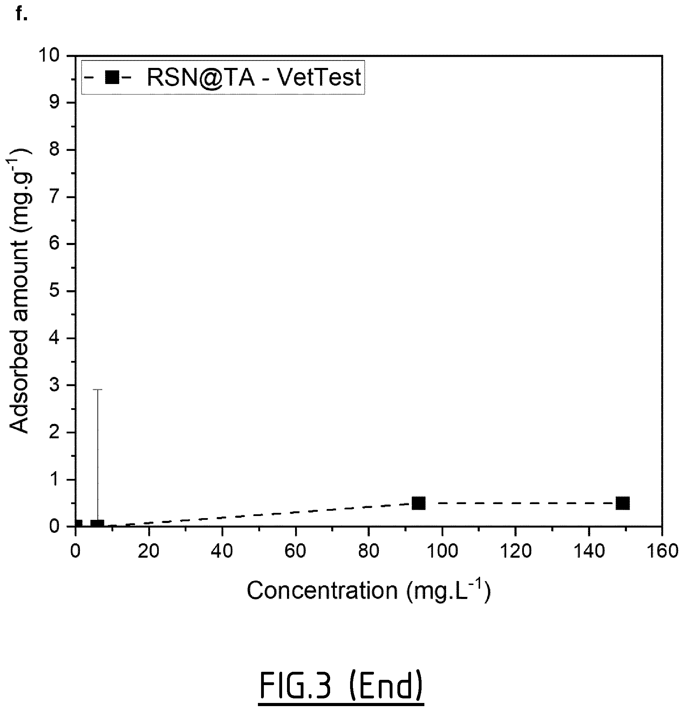

Adsorption experiments involving uremic toxins - creatinine, urea, indoxyl sulfate, p-cresol sulfate - and beneficial compounds - glucose and calcium - were performed to evaluate RSN and RSN@TA adsorption capacity on other compounds present in blood or in dialysate. All experiments were performed at room temperature, with suspensions of materials at fixed quantity of 1000 mg.L⁻¹ in contact during 4 h with adsorbates at various concentrations and at pH 7. It can be seen on figures 3.a-f. that none of these compounds were adsorbed on the surface or RSN or RSN@TA except for glucose where 5% were adsorbed on RSN@TA at relatively high concentration (3000 mg.L⁻¹).

### Example 4: Phosphate diffusion studies

### 4.1. Calibration

In order to confirm the relevance of the proposed experimental set-ups for studying phosphate diffusion, preliminary experiments in the absence of material were carried out.

### 4.1.1. Validation of the choice of membrane porosity.

The porosity of the membrane was chosen to reproduce the apparent permeability of the peritoneal membrane, i.e. the effective diffusion only of small water soluble and middle molecules.

Thus, molecules of methylthioninium chloride ("MB", approx. 300 Da, small water-soluble like), fluorophore-labeled glucose ("marked glucose", approx. 400 Da, middle molecule like) and fluorescein isothiocyanate labelled bovine serum albumin ("BSA-FITC", approx. 66 kDa, protein-bound molecule like) were involved in cassette mode-phosphate diffusion experiments as molecules of different molecular weight. All experiments were performed with ultrapure water in dialysate and blood compartment, at room temperature. Quantification of MB was performed by UV-VIS spectroscopy while marked glucose and BSA-FITC were quantified by fluorescence spectroscopy.

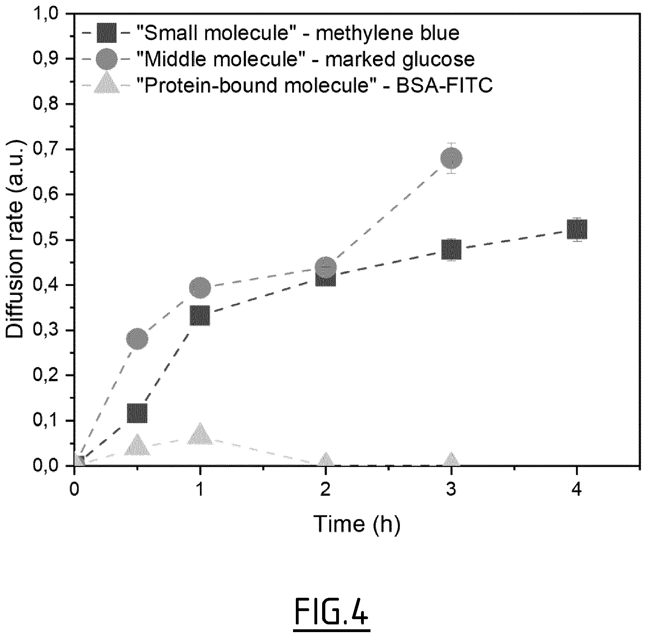

By sampling in the blood compartment, it has been observed that both MB and marked glucose concentration decrease over time and thus effectively diffuse through the membrane. 70% of the initial concentration of MB and 52% of the initial concentration of marked glucose were transported out of blood compartment. In addition, BSA-FITC concentration remained constant, confirming its retention in the blood compartment by the membrane (Figure 4).

### 4.1.2. Influence of dialysis and dialysate solutions on phosphate diffusion.

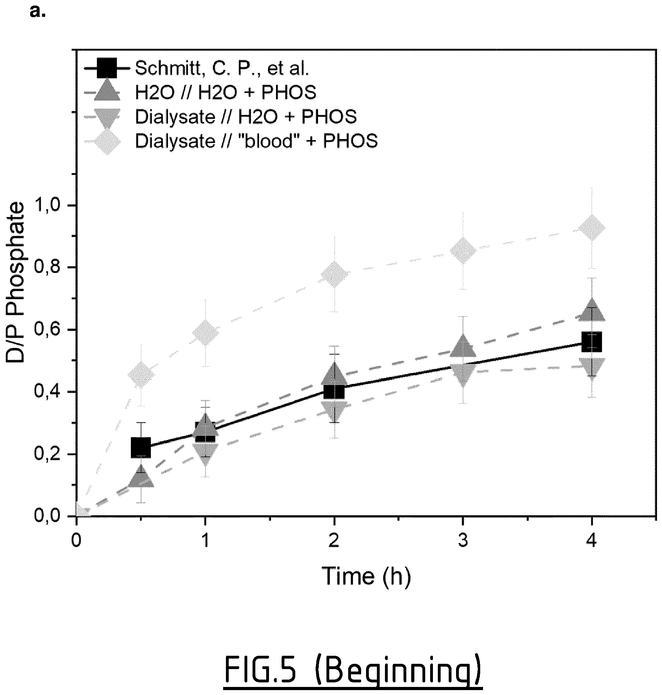

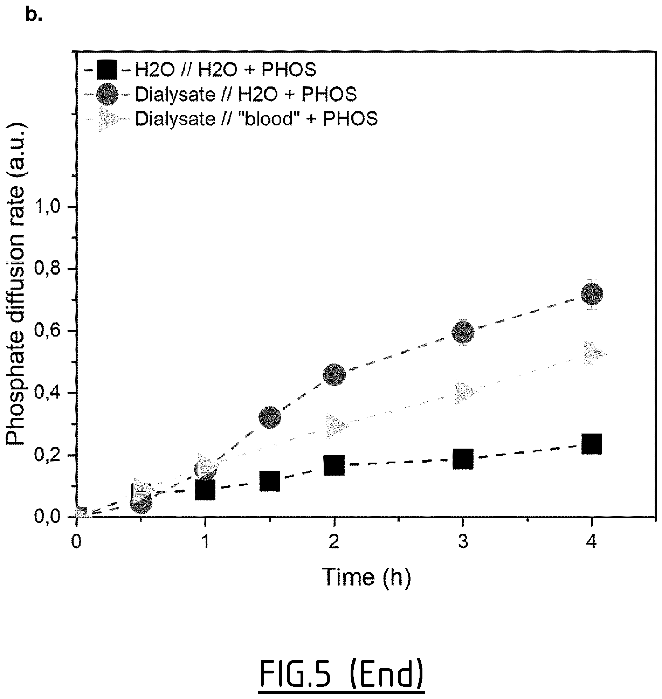

Experiments in tubing and cassette mode were performed with solutions of increasing complexity. First, ultrapure water containing phosphates in blood compartment at concentration 50 P-mg.L⁻¹ against ultrapure water in dialysate compartment was performed. Then, ultrapure water from dialysate compartment was replaced with a dialysate solution BICAVERA 1.5% GLUCOSE. Finally, ultrapure water in the blood compartment was replaced with the pseudo-blood solution (Figures 5.a-b)

In tubing experiments whose volume ratio blood/dialysate configuration is similar to reality; phosphate diffusion kinetic values were compared with reference values measured by Schmitt et al. in the blood of a male adult undergoing a PD process. Phosphate diffusion kinetic behaviors for the experiments involving ultrapure water in both compartment and ultrapure water against dialysate in dialysate compartment were very similar to the reference. When ultrapure water was replaced with pseudo-blood solution against dialysate, phosphate diffusion was both significantly accelerated and increased in intensity. Thus, the closeness of the experimental values to the reference tend to validate the tubing set-up for mimicking PD process.

For cassette mode configuration, reference was built by performing an experiment with ultrapure water in dialysate compartment against phosphate solution in ultrapure water at concentration 50 P-mg.L⁻¹ in blood compartment. Moving from ultrapure water to dialysate in dialysate compartment then pseudo-blood solution in blood compartment both significantly accelerated and increased phosphate diffusion kinetics and intensity.

### 4.1.3. Influence of temperature and stirring as parameters similar to physiological conditions on phosphate diffusion.

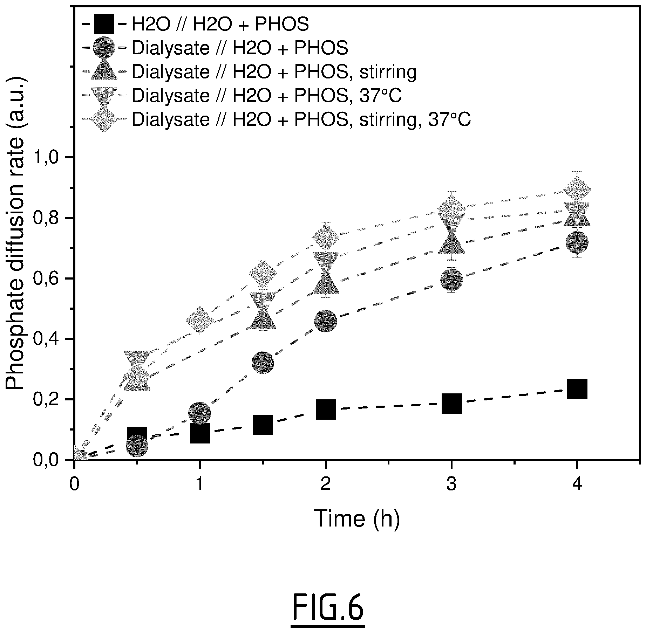

Experiment respectively at 37°C to mimic body temperature; with slow mechanical stirring in dialysate compartment to reproduce the peristaltic movement and a combination of these two parameters were achieved and compared to the previously established reference values. Both temperature and stirring allowed a slight increase in phosphate diffusion kinetics and intensity. Higher values of diffusion rate were found when combining temperature and stirring (Figure 6).

### 4.2. Phosphate diffusion experiments in tubing mode

Experiments in the presence of Iron Oxide Nanostructures coated with different surfactant were first performed in tubing mode and compared to reference experiment with ultrapure water as dialysate and dialysis solutions and without material in dialysate compartment. All the experiments were performed at room temperature, during 4 h.

Nanomaterials that showed the best performances were iron oxide nanoparticles obtained by coprecipitation method and coated with the cationic polymer PDADMAC (IO-CP+PDADMAC).

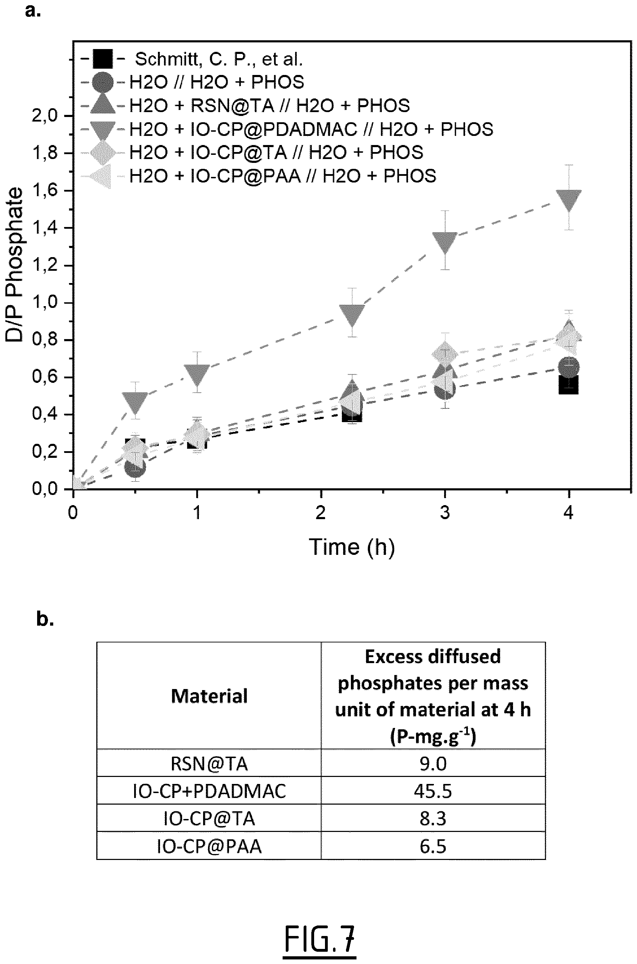

In experiments involving iron oxide nanostructures coated with anionic surfactant - Raspberry-Shaped Nanostructures obtained by polyol solvothermal method and coated with tannic acid (RSN@TA); Iron Oxide Nanoparticles obtained by coprecipitation and coated with tannic acid and poly(acrylic) acid (IO-CP@PAA) - phosphate diffusion intensity has also been increased (Fig. 7.a). The excess quantity of diffused phosphates due to the presence of IONS in comparison with reference experimental value in ultrapure water has been calculated and summarized in figure 7.b.

### 4.3. Phosphate diffusion experiments in cassette mode

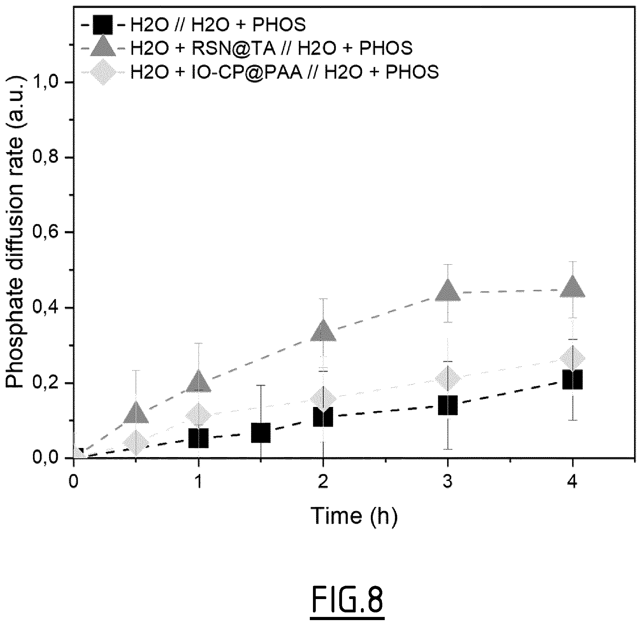

Experiments in the presence of iron oxide nanostructures coated with different surfactant were secondly performed in cassette mode and compared to reference experiment with ultrapure water as dialysate and dialysis solutions with and without material in dialysate compartment. In figure 8, has been compared the diffusion rate of phosphate in the presence of RSN@TA and IO-CP@TA. The introduction of both nanomaterials allowed to enhance the phosphate diffusion rate.

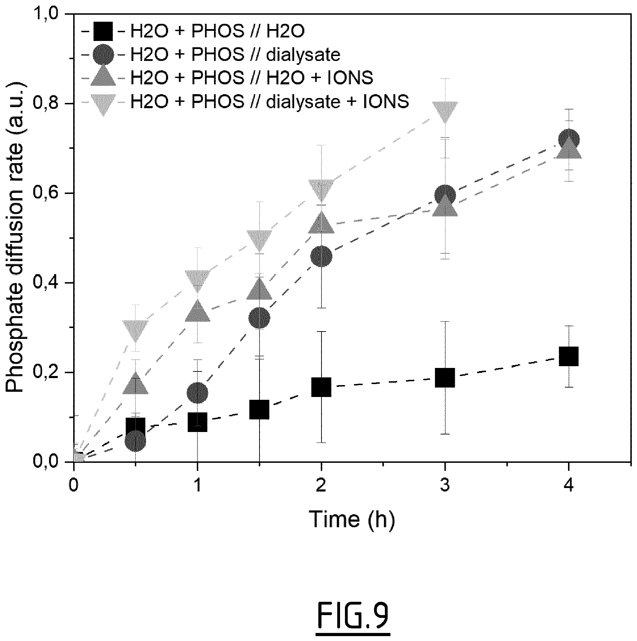

Experiments in the presence of iron oxide nanoparticles coated with tannic acid was secondly performed in cassette mode and compared to reference experiment with ultrapure water or dialysate, without material in dialysate compartment. All experiments were performed at room temperature. The introduction of materials allowed to enhance the phosphate diffusion rate in comparison with reference experiments, with a maximum diffusion rate for dialysate compartment filled with IO-CP@TA in dialysate (figure 9).

## Claims

1. A dialysate composition having a composition allowing for a balance with blood, said composition comprising mineral salts and a pH buffer, and comprising at least one iron oxide nanoparticle (Fe²⁺ and/or Fe³⁺) coated with at least one polymeric compound.

2. The dialysate composition according to claim 1, which further comprises at least one osmotic compound.

3. The dialysate composition according to claim 1 or 2, wherein the iron oxide nanoparticle is coated with one polymeric compound or mixture of polymeric compounds.

4. The dialysate composition according to claim 1 to 3, wherein the iron oxide nanoparticle is coated with a polymeric compound selected from the group consisting of tannic acid, carboxymethyl dextran, polyacrylic acid, PDAD, PEI, PAH and mixtures thereof.

5. The dialysate composition according to any one of the claims 1 to 4, wherein the iron oxide nanoparticle has a molar ratio polymeric: iron (i.e. polymer: Fe) ranging from 0.001 to 5, preferably from 0.01 to 2.

6. The dialysate composition according to any one of the claims 1 to 5, wherein the iron nanoparticle has an average hydrodynamic diameter ranging from 10 nm and 300 nm, preferably from 20 nm and 200 nm, and more preferably from 30 nm and 150 nm.

7. The dialysate composition according to any one of the claims 1 to 6, wherein the pH is comprised between 4 and 8, preferably between 6 and 8 and more preferably between 6.5 and 7.

8. The dialysate composition according to any one of the claims 1 to 7, wherein the concentration of iron oxide nanoparticles in said dialysate composition is from 0.04 mmol.L⁻¹ to 4 mol.L⁻¹, preferably from 0.04 mmol.L⁻¹ to 0.4 mol.L⁻¹, and even more preferably from 0.04 mmol.L⁻¹ to 20 mmol.L⁻¹.

9. The dialysate composition according to any one of the claims 1 to 8, wherein said dialysate composition is a composition for hemodialysis.

10. The dialysate composition according to claim 9, comprising:
(a) at least one pH buffer,
(b) at least one mineral salt, and
(c) at least one iron oxide nanoparticle (Fe²⁺ and/or Fe³⁺) coated with at least one polymeric compound.

11. The dialysate composition according to any one of the claims 1 to 8, wherein said dialysate composition is a composition for peritoneal dialysis.

12. The dialysate composition according to claim 11 comprising:
(a) at least one pH buffer,
(b) at least one mineral salt,
(c) at least one osmotic agent, preferably selected form the group consisting of glucose, icodextrin and mixtures thereof; and
(d) at least one iron oxide nanoparticle (Fe²⁺ and/or Fe³⁺) coated with at least one polymeric compound.

13. The dialysate composition according to any one of the claims 1 to 12, further comprising another nanoparticle selected from the groups consisting of inorganic nanoparticles, polymeric nanoparticles, polymeric capsules, organic-inorganic hybrid nanoparticles and organic-inorganic hybrid capsules.

14. The composition according to any one of the claims 1 to 13, for use as a medicament.

15. The composition according to any one of the claims 1 to 13, for use as a dialysate for blood purification or biological fluid purification.
